(19) Europäisches Patentamt
European Patent Office
Office européen des brevets

(11) Publication number: **0 340 754 B1**

(12) **EUROPEAN PATENT SPECIFICATION**

(45) Date of publication of patent specification: **20.07.94**

(51) Int. Cl.5: **C07C 409/24**, C07C 409/40, C11D 3/395, C11D 3/39

(21) Application number: **89108021.0**

(22) Date of filing: **03.05.89**

(54) **Monopersulfates of amino-(poly) percarboxylic acids.**

(30) Priority: **04.05.88 IT 2045588**

(43) Date of publication of application:
**08.11.89 Bulletin 89/45**

(45) Publication of the grant of the patent:
**20.07.94 Bulletin 94/29**

(84) Designated Contracting States:
**AT BE CH DE ES FR GB LI NL SE**

(56) References cited:
**EP-A- 0 166 571**
**EP-A- 0 170 386**
**EP-A- 0 316 809**
**EP-A- 0 340 755**

**CHEMICAL ABSTRACTS, vol. 105, no. 22,1 December 1986, page 133, abstract no. 193384w, Columbus, Ohio, US; & JP-A-6181499**

(73) Proprietor: **AUSIMONT S.p.A.**
**Foro Buonaparte, 31**
**I-20121 Milano(IT)**

(72) Inventor: **Venturello, Carlo**
**135, via XXIII Marzo**
**I-28100 Novara(IT)**
Inventor: **Cavallotti, Claudio**
**3, Via Lorenteggio**
**I-20100 Milan(IT)**
Inventor: **Achilli, Fiorella**
**6, Strada Pianello**
**I-29010 Agazzano Piacenza(IT)**

(74) Representative: **Weinhold, Peter, Dr. et al**
**Patentanwälte Dr. V. Schmied-Kowarzik**
**Dipl.-Ing. G. Dannenberg**
**Dr. P. Weinhold**
**Dr. D. Gudel**
**Dipl.-Ing. S. Schubert**
**Dr. P.**
**Barz**
**Siegfriedstrasse 8**
**D-80803 München (DE)**

**EP 0 340 754 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to new derivatives of (poly)percarboxylic acids, which can be referred to as monopersulfates of amino-(poly)percarboxylic acids, to a process for the preparation of said compounds and to their use in bleaching compositions.

More particularly, the present invention relates to monopersulfates of general formula (I):

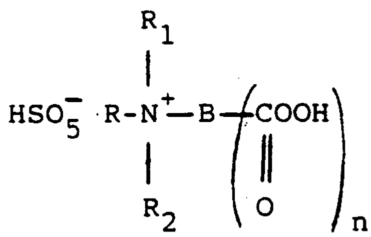

(I)

wherein:

R, $R_1$ and $R_2$, the same or different from each other, are selected from hydrogen and alkyl groups which may optionally be substituted, provided that at least one of said groups is a hydrogen atom;

B is an alkylene group which may optionally have one or more interspersed $-CONR_3-$ groups, wherein $R_3$ represents a hydrogen atom or an alkyl or aryl group; and

n is an integer of from 1 to 6.

The derivatives of general formula (I) constitute a new class of products highly interesting from an industrial point of view, particularly due to their high content of active oxygen per weight unit.

In fact, they can find general use, similarly to already known peracids, e.g. in the field of plastics, as polymerization initiators and, in particular, as oxidants for olefin epoxidation and hydroxylation, and in many other oxidative processes in the field of fine chemistry.

Particularly interesting and efficient, however, is their application in the field of bleaching, i.e. in detergents.

In the past years, organic peracids have attracted increasing interest in the industrial field due to their excellent properties when used as bleaching agents in compositions for medium/low temperature washing, and particularly in view of energy saving considerations (EP-A-0 166 571, EP-A-0 170 386).

Consequently, there is considerable research activity aiming at finding new organic peracid compounds having the necessary properties, i.e. high bleaching activity, thermal stability and storage stability or shelf life. The latter requirements are essential for an industrial application and a widespread use of such compounds.

Therefore, many mono- and di-percarboxylic acids, straight-chained or cyclic, are know and used, among others, in the field of detergence.

Already described percarboxylic acids are, e.g.: diperdodecanedioic acid, monoperphthalic acid, diperazelaic acid and substituted diperglutaric and-adipic acids, etc.

In conventional percarboxylation processes the oxidation of the substrate is carried out in concentrated $H_2SO_4$ with a solution of hydrogen peroxide.

The above method, when applied to substrates containing salifiable nitrogen atoms of basic character, results in products with a high solubility in the strongly acidic medium.

Said high solubility makes it impossible to apply any of the conventional processes of isolation of the percarboxylic acid derivative which may be formed, such as precipitation and extraction with an organic solvent.

Surprisingly, it has now been found that the amino-(poly)percarboxylic acid monopersulfates of general formula (I), salified on the nitrogen atom with the persulfuric anion, can be obtained, according to the invention, in a stable form by means of a novel process, which is also an object of the present invention.

Therefore, one object of the present invention is to provide novel compounds, i.e. monopersulfates of amino-(poly)percarboxylic acids having the above formula (I).

Another object of the present invention is to provide a simple and inexpensive process for the preparation of the percarboxylic acid derivatives having the above formula (I) in stable form.

A further object of the present invention is the use of the amino-percarboxylic acid monopersulfates of formula (I) as bleaching agents in detergent formulations, particularly those for low-medium temperature use.

These, and still other objects, which will become evident from the following detailed disclosure, are achieved, according to the present invention, by the monopersulfates of amino-(poly)percarboxylic acids of the above formula (I), and a process for the preparation thereof, which is characterized in that a substrate selected from amino-(poly)carboxylic acids or their N-sulfate salts, said substrate corresponding to the desired monopersulfate of formula (I) is reacted with $H_2O_2$ in concentrated $H_2SO_4$ and in that said monopersulfate is then separated from the reaction mixture by means of the addition of an organic solvent selected from tetrahydrofuran and ethyl acetate and mixtures thereof.

By use of the present process, the monopersulfates of formula (I) are generally obtained as stable solids, salified on the nitrogen atom with the $HSO_5$ anion, due to their insolubilization in the reaction medium by the solvent.

Stated more precisely, the process according to the present invention comprises the percarboxylation reaction of a substrate selected from an amino-(poly)carboxylic acid, or its N-sulfate, corresponding to the desired salt of formula (I), in an acidic medium of concentrated $H_2SO_4$, with $H_2O_2$ and the subsequent addition, at the end of the reaction, of a suitable organic solvent which is immiscible with the desired product(by not dissolving it), but which is capable, on the contrary, of completely dissolving the acid reaction medium (concentrated $H_2SO_4$), as well as the excess of $H_2O_2$ and the reaction water. This results in the separation of the desired monopersulfate product of formula (I) in a stable solid form.

As stated above, the substrate used as starting material corresponds in structure to the desired compound of formula (I) and is selected from amino-(poly)carboxylic acids, optionally already salified as sulfate on the nitrogen atom.

The substrates used as the starting material are known compounds, and/or can be prepared according to conventional techniques.

The obtained product may then be filtered, washed with the solvent, dried, in the usual manner.

With reference to formula (I) R, $R_1$ and $R_2$ may be identical or different groups. They may represent hydrogen or linear or branched alkyl groups, preferably containing 1 to 10, and particularly 1 to 5, carbon atoms. Examples of such groups are methyl, ethyl, n-propyl, i-propyl, n-butyl, i-butyl, sec.-butyl, ter.-butyl, pentyl, i-pentyl, hexyl, octyl and decyl.

The above alkyl groups may optionally be substituted by one or more, preferably 1 to 3, substituents. If several substituents are present they may be the same or different. Said substituents should be inert under the reaction conditions of the peroxydation and also inert towards the resulting percarboxylic acid. Suitable and preferred substituents are halogen atoms, i.e. F, Cl, Br and I, particularly F and Cl, OH, $NO_2$, lower alkoxy groups, i.e. alkoxy groups having 1 to 6, preferably 1 to 4, carbon atoms, such as, e.g. methoxy, ethoxy, propoxy, i-propoxy and butoxy, carboxylic groups ($COOR_4$, $OCOR_4$, $COR_4$ wherein $R_4$ is preferably hydrogen or $C_{1-10}$ alkyl).

B represents a (preferably) linear or a branched alkylene group $(CH_2)_m$ wherein m preferably is an integer of from 1 to 20, particularly from 6 to 18, such as the alkylene groups derived from the above mentioned alkyl groups (methylene, ethylene, propylene, butylene, hexylene, octylene, decylene, undecylene, duodecylene, tridecylene, pentadecylene, heptadecylene, octadecylene).

The group B may optionally carry one or several substituents, preferably 1 to 3. Examples thereof are those already given in connection with the groups R, $R_1$ and $R_2$. The group B also may optionally have interspersed $CONR_3$ units (preferably 1 to 3 such units), wherein $R_3$ is hydrogen, (preferably $C_{1-5}$-alkyl (e.g. the alkyl groups mentioned above) or aryl (preferably having 6 to 14 carbon atoms, e.g. phenyl).

n in formula (I) is an integer of from 1 to 6, preferably 1 to 3. Particularly preferred are compounds wherein n is 1 or 2.

Esters which may also optionally be used as starting materials for obtaining the compounds of formula (I) are preferably the $C_{1-12}$ alkyl esters, e.g. the methyl, ethyl, propyl or butyl esters.

According to a preferred mode of operation, the percarboxylation reaction of the amino-(poly)carboxylic acids or the corresponding N-sulfates used as starting material is carried out by gradually adding $H_2O_2$ having a concentration within the range of from 70% to 90% by weight to a solution of the substrate in concentrated $H_2SO_4$ (96 - 98%), maintaining the reaction temperature throughout the reaction within a range of from 0 °C to 20 °C.

Alternatively, it has been found that it is possible to salify (as $H_2SO_4$ salt) the substrate by working in the absence of $H_2O_2$ under otherwise the same conditions as described above, and to separate the obtained salt first, said salt being then peroxidized.

The amount of $H_2SO_4$, based on a concentration of 100%, is preferably at least about 5 moles per mole of substrate, and most preferred ranges are from 6 to 7 moles.

Hydrogen peroxide is preferably used in an amount which is in excess with respect to the substrate, and preferably is present in amounts of at least 6 moles per each mole of substrate, particularly of from 7 to

10 moles per mole of substrate.

The reaction time depends on the nature of the substrate, the operating temperature, and on the final total $H_2SO_4/H_20$ molar ratio present at the end of the reaction. Said ratio is preferably chosen so as to be of from 1.2 to 1.4 by adjusting the various parameters concerned.

Reaction times of from 30 minutes to 2 hours have proven to be the usual ones.

The employed amount of tetrahydrofuran and/or ethyl acetate solvent is preferably not lower than 4 litres/mole of substrate, e.g., 5 litres/mole; furthermore, the solvent is preferably added at a temperature of not higher than about 10 °C.

The amino-percarboxylic acid monopersulfates of formula (I) usually are solid at room temperature. They can be used to particular advantage in formulations of detergent compositions, e.g., granular formulations, and as bleaching agents in solution within a wide temperature range.

The detergent compositions can be formulated according to the usual pertinent techniques, using further components and/or additives.

The present invention will now be described in more detail by the following examples, which are given for purely illustrative purposes.

The products prepared in the examples were characterized by elemental analysis, by determining their content of active oxygen (by iodometric titration), and by using Fourier Transform Infrared Spectroscopy (FT-IR).

EXAMPLE 1

4 g (0.0164 mole) of N, N-dimethylaminolauric acid were added, slowly and under stirring, to 2.5 g of sulfuric acid in a 25 ml beaker, care being taken to maintain the temperature below or at 40 °C by use of a cooling bath. Stirring was continued at 35 to 40 °C for 1 hour, up to complete dissolution.

The reaction mixture was then poured into 150 ml of ethyl acetate maintained under stirring at 10 °C. Stirring was then continued for 30 minutes.

The separated N, N-dimethylaminolauric acid sulfate was filtered over a porous septum, consecutively washed with ethyl acetate (2 x 30 ml) and $Et_20$ (3 x 30 ml), and then dried under vacuum at room temperature over $CaCl_2$.

4.4 g of product were obtained, which were used for preparing the corresponding peracid monopersulfate.

3.6 g of $H_2SO_4$ (96%, 0.0352 mole) were placed in a beaker equipped with stirrer, thermometer and external bath.

2 g of N, N-dimethylaminolauric acid sulfate (0.0059 mole) were added under stirring at 15 °C. 2.2 g of $H_2O_2$ (85%, 0.055 mole) were then added so as to not exceed a temperature of +15 °C.

Stirring was continued at +15 °C for 1 hour. At the end, the reaction mixture was poured into 50 ml of ethyl acetate, maintained under stirring at -10 °C. After 30 minutes, the separated crystalline product was filtered over a porous septum (under vacuum) and thereafter was washed with 2 x 30 ml of ethyl acetate and then with 2 x 30 ml of ethyl ether.

Finally, the product was dried for 1 hour under vacuum, at room temperature in a Ca $Cl_2$ drier.

Obtained were 1.8 g of crystalline N, N-dimethylaminoperlauric acid monopersulfate having an active oxygen content of 8.42% (98.4% of the theoretical value).

Yield: 80%

Elemental Analysis

Calculated for $C_{14}H_{31}NSO_8$: C 45.02%; H 8.36%; N 3.75%; O (active) 8.56%; $H_2SO_5$ 30.54%.
Found: C 45.23%; H 8.58%; N 3.71%; O (active) 8.42%; $H_2SO_5$ 29.9%.
Melting point: 45 °C (with decomposition).

EXAMPLE 2

2.8 g of $H_20_2$ (85%, 0.07 mole) were added, under stirring, to 5.7 g of $H_2SO_4$ (96%, 0.056 mole), so as to maintain the temperature below +5 °C.

2 g of 12-aminolauric acid (0.0093 mole) were then added by maintaining the temperature at or below +15 °C. Stirring was continued at +15 °C for 1 hour. The reaction mixture was poured into 50 ml of ethyl acetate whereafter the procedure described in example 1 was followed.

Obtained were 3 g of crystalline 12-aminoperlauric acid monopersulfate having an active oxygen content of 8.95% (96.7% of the theoretical value).

Yield: 90%.

Elemental Analysis

Calculated for $C_{12}H_{27}NSO_8$: C 41.72%; H 7.88%; N 4.05%; 0 (active) 9.26%; $H_2SO_5$ 33.02%.
Found: C 41.69%; H 7.92%; N 4.03%; 0 (active) 8.95%; $H_2SO_5$ 33.01%.
Melting point: 73 °C (with decomposition).

EXAMPLE 3

6.8 g of $H_2O_2$ (85%, 0.17 mole) were added, under stirring, to 21.5 g of $H_2SO_4$ (96%, 0.211 mole) so as to maintain the temperature below + 5 °C. 2 g of p. alanine (0.0224 mole) were added, keeping the temperature at or below +15 °C. Stirring was then continued at +15 °C for 1 hour.

Thereafter, the reaction mixture was poured into 100 ml of ethyl acetate and the procedure of example 1 was then followed.

Obtained were 4.3 g of product having an active oxygen content of 12.5%, corresponding to about 80% of 3-aminoperpropionic acid monopersulfate.

Elemental Analysis

Calculated for $C_3H_9NSO_8$: 0 (active) 14.6%; $H_2SO_5$ 52.05%; total S 14.6%.
Found: 0 (active) 12.5%; $H_2SO_5$ 41.60%; total S 14.78%.
Melting point: 82 °C (with decomposition).

EXAMPLE 4 (Application Example)

Bleaching tests were carried out with the novel salts listed in the annexed Tables 1 and 2, at alkaline pH (Table 1) and acid pH (Table 2). The following substance served as comparison:
H 48 (Mg salt of monoperphthalic acid), a commercial peracid known in the detergent art, and manufactured by INTEROX Chemical Ltd., London, U.K. (Tables 1 and 2).

All tests were carried out at constant temperature (60 °C), with an initial concentration of total active oxygen in the bleaching solution (identical for all products) of equal to 200 mg/l.

Process

For each test, 500 ml of deionized water, contained in a 1000 ml flask equipped with a condenser, was heated to a temperature of 60 °C and adjusted to a pH of 9.5 (by adding a few drops of an NaOH solution) (Table 1) and of 3 - 4 (by adding a few drops of diluted $H_2SO_4$)(Table 2). Then the bleaching product was added with stirring, in amounts shown in the following Tables, and immediately thereafter two cotton specimens of 10 cm x 10 cm, stained with standard stains of red wine at EMPA INSTITUTE of St.Gallen (Switzerland), and marked with the "EMPA 114" mark, were added.

The system subsequently was kept under stirring for 60 minutes and, at the end of this time, the specimens, rinsed under running water, were dried and ironed, and were then subjected to an evaluation of the bleaching effect by means of a measurement of the whiteness degree by reflectometry. The results are reported in the following Tables 1 and 2, wherein the data are expressed as % Bleaching, defined as:

$$\% \text{ Bleaching} = \frac{A - B}{C - B} \times 100\%$$

wherein:

A = degree of whiteness (%) of the specimen bleached after the test;
B = degree of whiteness (%) of the specimen before the test;
C = degree of whiteness (%) of the completely bleached specimen.

The degree of whiteness was measured by means of an Elrepho Zeiss reflectometer, assuming MgO = 100% of whiteness, and using a filter N.6 (λ = 464 nm).

The data listed in Table 1, concerning tests carried out at alkaline pH, show that the peracid salts of the present invention have a bleaching power which may be compared to the bleaching power of H 48.

Likewise, the results, expressed as % Bleaching, listed in Table 2, show that the tested products have a bleaching power in acid solution which is particularly high and higher than that of H 48.

This is particularly surprising in consideration of the fact that the peroxidic compounds generally show a very modest and sometimes negligible bleaching activity at acid pH.

TABLE 1

| Test carried out at alkaline pH (9.5) | | | |
|---|---|---|---|
| Compound | Amounts used in the tests (grams) | Initial concentration of total active oxygen (mg/l) | % Bleaching |
| - Example 1 (titer = 8.42% of active oxygen) | 1.19 | 200 | 80.2 |
| - H 48 (titer = 5.5% of active oxygen) | 1.86 | 200 | 81.0 |

TABLE 2

| Test carried out at acid pH (3-4) | | | |
|---|---|---|---|
| Compound | Amounts used in the tests (grams) | Initial concentration of total active oxygen (mg/l) | % Bleaching |
| - Example 1 (titer = 8.42% of active oxygen) | 1.19 | 200 | 78.2 |
| - Example 2 (titer = 8.95% of active oxygen) | 1.12 | 200 | 78.9 |
| - H 48 (titer = 5.5% of active oxygen) | 1.86 | 200 | 60.0 |

## Claims

**Claims for the following Contracting States : AT, BE, CH, DE, FR, GB, LI, NL, SE**

**1.** Monopersulfates of amino-(poly)percarboxylic acids of the general formula:

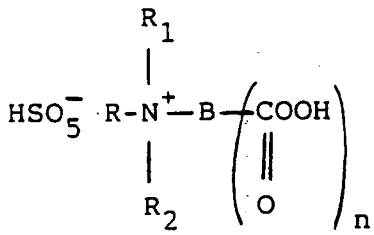

(I)

wherein:

R, $R_1$ and $R_2$, the same or different from each other, are selected from hydrogen and optionally substituted alkyl groups, provided that at least one of R, $R_1$ and $R_2$ is a hydrogen atom;

n is an integer of from 1 to 6 and B is an alkylene group optionally having interspersed -$CONR_3$-units, wherein $R_3$ is a hydrogen atom or an alkyl or aryl group.

**2.** Monopersulfates according to claim 1, wherein R, $R_1$ and $R_2$ are selected from hydrogen or linear or branched $C_{1-10}$ alkyl groups and the alkylene group B has from 6 to 18 carbon atoms.

**3.** Monopersulfates according to anyone of claims 1 and 2, wherein the alkylene groups B have at least one interspersed $CONR_3$-unit wherein $R_3$ represents a hydrogen atom, a $C_{1-5}$ alkyl group or an aryl group.

**4.** Monopersulfates according to claim 1, i.e. 12-(N,N-dimethyl-amino)perlauric acid monopersulfate, 12-amino-perlauric acid monopersulfate, and 3-amino-perpropionic acid monopersulfate.

**5.** Process for preparing the monopersulfates according to claim 1, characterized in that a substrate selected from an amino-(poly)carboxylic acid and its N-sulfate salt, corresponding to the desired monopersulfate of general formula (I), is reacted with $H_2O_2$ in a concentrated $H_2SO_4$ medium and that the resulting monopersulfate of general formula (I) is separated from the reaction mixture by addition of tetrahydrofuran and/or ethyl acetate.

**6.** Process according to claim 5, characterized in that an amino-(poly)carboxylic acid corresponding to the desired compound of formula (I) is converted into the corresponding $HSO_4$ salt which is then reacted with $H_2O_2$ in concentrated $H_2SO_4$.

**7.** Process according to any one of claims 5 and 6, characterized in that the amino-(poly)carboxylic acid or its N-sulfate salt is gradually reacted with $H_2O_2$ having a concentration of from 70% to 90% by weight in concentrated $H_2SO_4$ at a temperature of from 0° to 20°C.

**8.** Process according to any one of claims 5 to 7, characterized in that the amount of $H_2SO_4$ is at least equal to about 5 moles, and preferably ranges from 6 to 7 moles, per mole of substrate.

**9.** Process according to any one of claims 5 to 8, characterized in that the hydrogen peroxide is used in an amount of at least about 6 moles, and preferably of from 7 to 10 moles, per mole of substrate.

**10.** Process according to any one of claims 5 to 9, characterized in that the final molar ratio of $H_2SO_4$ to the total $H_2O$ present at the end of the reaction ranges from 1.2 to 1.4.

**11.** Process according to any one of claims 5 to 10, characterized in that the amount of tetrahydrofuran and/or ethyl acetate used is not lower than about 4 litres per mole of substrate.

**12.** Process according to any one of claims 5 to 11, characterized in that tetrahydrofuran and/or ethyl acetate solvent is added at a temperature not higher than about 10°C.

**13.** Use of the monopersulfates according to any one of claims 1 to 4 as bleaching agents in detergent formulations.

**Claims for the following Contracting State : ES**

**1.** Process for preparing monopersulfates of amino(poly)percarboxylic acids of the general formula:

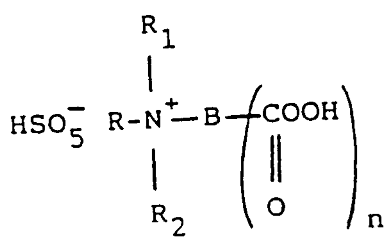

(I)

wherein:

R, $R_1$ and $R_2$, the same or different from each other, are selected from hydrogen and optionally substituted alkyl groups, provided that at least one of R, $R_1$ and $R_2$ is a hydrogen atom;

n is an integer of from 1 to 6 and B is an alkylene group optionally having interspersed -$CONR_3$-units, wherein $R_3$ is a hydrogen atom or an alkyl or aryl group,

characterized in that a substrate selected from an amino(poly)carboxylic acid and its N-sulfate salt, corresponding to the desired monopersulfate of general formula (I), is reacted with $H_2O_2$ in a concentrated $H_2SO_4$ medium and that the resulting monopersulfate of general formula (I) is separated from the reaction mixture by addition of tetrahydrofuran and/or ethyl acetate.

2. Process according to claim 1, wherein R, $R_1$ and $R_2$ are selected from hydrogen or linear or branched $C_{1-10}$ alkyl groups and the alkylene Group B has from 6 to 18 carbon atoms.

3. Process according to any one of claims 1 and 2, wherein the alkylene groups B have at least one interspersed $CONR_3$-unit wherein $R_3$ represents a hydrogen atom, a $C_{1-5}$ alkyl group or an aryl group.

4. Process according to claim 1, wherein as monopersulfate of formula (I) 12-(N,N-dimethyl-amino)-perlauric acid monopersulfate, 12-amino-perlauric acid monopersulfate or 3-amino-perpropionic acid monopersulfate is prepared.

5. Process according to any one of claims 1 to 4, characterized in that an amino-(poly)carboxylic acid corresponding to the desired compound of formula (I) is converted into the corresponding $HSO_4$ salt which is then reacted with $H_2O_2$ in concentrated $H_2SO_4$.

6. Process according to any one of claims 1 to 5, characterized in that the amino-(poly)carboxylic acid or its N-sulfate salt is gradually reacted with $H_2O_2$ having a concentration of from 70% to 90% by weight in concentrated $H_2SO_4$ at a temperature of from 0° to 20°C.

7. Process according to any one of claims 1 to 6, characterized in that the amount of $H_2SO_4$ is at least equal to about 5 moles, and preferably ranges from 6 to 7 moles, per mole of substrate.

8. Process according to any One of claims 1 to 7, characterized in that the hydrogen peroxide is used in an amount of at least about 6 moles, and preferably of from 7 to 10 moles, per mole of substrate.

9. Process according to any one of claims 1 to 8, characterized in that the final molar ratio of $H_2SO_4$ to the total $H_2O$ present at the end of the reaction ranges from 1.2 to 1.4.

10. Process according to any one of claims 1 to 9, characterized in that the amount of tetrahydrofuran and/or ethyl acetate used is not lower than about 4 litres per mole of substrate.

11. Process according to any one of claims 1 to 10, characterized in that tetrahydrofuran and/or ethyl acetate solvent is added at a temperature not higher than about 10°C.

12. Use of the monopersulfates according to any one of claims 1 to 4 as bleaching agents in detergent formulations.

**Patentansprüche**

**Patentansprüche für folgende Vertragsstaaten: AT, BE, CH, DE, FR, GB, LI, NL, SE**

1. Monopersulfate von Amino-(poly)percarbonsäuren der allgemeinen Formel:

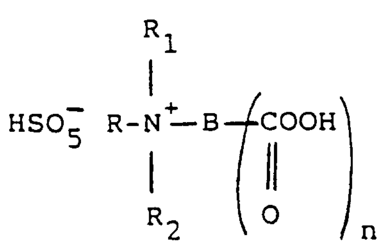

(I)

wobei
R, $R_1$ und $R_2$, die gleich oder von einander verschieden sein können, aus Wasserstoff und wahlweise substituierten Alkylgruppen ausgewählt sind, vorausgesetzt, daß mindestens einer der Reste R, $R_1$ und $R_2$ ein Wasserstoffatom ist;
n eine ganze Zahl von 1 bis 6 bedeutet und B eine Alkylengruppe ist, die gegebenenfalls eingeschobene -$CONR_3$-Einheiten aufweist, wobei $R_3$ ein Wasserstoffatom oder eine Alkyl- oder eine Arylgruppe ist.

2. Monopersulfate gemäß Anspruch 1, wobei R, $R_1$ und $R_2$ aus Wasserstoff oder linearen oder verzweigten $c_{1-10}$ Alkylgruppen ausgewählt sind und die Alkylengruppe B von 6 bis 18 Kohlenstoffatome besitzt.

3. Monopersulfate gemäß irgendeinem der Ansprüche 1 und 2, wobei die Alkylengruppe B mindestens eine eingeschobene $CONR_3$-Einheit aufweist, wobei $R_3$ für ein Wasserstoffatom, eine $C_{1-5}$ Alkylgruppe oder eine Arylgruppe steht.

4. Monopersulfate gemäß Anspruch 1, und zwar 12-(N,N-Dimethylamino)perlaurinsäure-monopersulfat, 12-Amino-perlaurinsäure-monopersulfat und 3-Amino-perpropionsäure-monopersulfat.

5. Verfahren zur Herstellung der Monopersulfate gemäß Anspruch 1, dadurch gekennzeichnet, daß ein Substrat, das ausgewählt ist aus einer Amino-(poly)carbonsäure und deren N-Sulfat-Salz, die dem gewünschten Monopersulfat der allgemeinen Formel (I) entsprechen, mit $H_2O_2$ in einem Medium aus konzentrierter $H_2SO_4$ umgesetzt wird und daß das daraus resultierende Monopersulfat der allgemeinen Formel (I) durch Zugabe von Tetrahydrofuran und/oder Ethylacetat von der Reaktionsmischung abgetrennt wird.

6. Verfahren gemäß Anspruch 5, dadurch gekennzeichnet, daß eine Amino-(poly)carbonsäure, die der gewünschten Verbindung der Formel (I) entspricht, in das entsprechende $HSO_4$-Salz umgewandelt wird, welches anschließend mit $H_2O_2$ in konzentrierter $H_2SO_4$ umgesetzt wird.

7. Verfahren gemäß irgendeinem der Ansprüche 5 und 6, dadurch gekennzeichnet, daß die Amino-(poly)-carbonsäure oder ihr N-Sulfat-Salz schrittweise mit $H_2O_2$ einer Konzentration von 70 Gew.-% bis 90 Gew.-% in konzentrierter $H_2SO_4$ bei einer Temperatur von 0° bis 20°C umgesetzt wird.

8. Verfahren gemäß irgendeinem der Ansprüche 5 bis 7, dadurch gekennzeichnet, daß die Menge an $H_2SO_4$ mindestens etwa 5 Mol, vorzugsweise von 6 bis 7 Mol, pro Mol Substrat beträgt.

9. Verfahren gemäß irgendeinem der Ansprüche 5 bis 8, dadurch gekennzeichnet, daß mindestens etwa 6 Mol Wasserstoffperoxid, vorzugsweise von 7 bis 10 Mol, pro Mol Substrat verwendet werden.

10. Verfahren gemäß irgendeinem der Ansprüche 5 bis 9, dadurch gekennzeichnet, daß das endgültige Mol-Verhältnis von $H_2SO_4$ zum gesamten am Ende der Reaktion anwesenden $H_2O$ im Bereich von 1,2 bis 1,4 liegt.

11. Verfahren gemäß irgendeinem der Ansprüche 5 bis 10, dadurch gekennzeichnet, daß die Menge des verwendeten Tetrahydrofurans und/oder Ethylacetats mindestens etwa 4 Liter pro Mol Substrat beträgt.

12. Verfahren gemäß irgendeinem der Ansprüche 5 bis 11, dadurch gekennzeichnet, daß das Tetrahydrofuran und/oder Ethylacetat bei einer Temperatur, die 10°C nicht übersteigt, zugegeben wird.

13. Verwendung der Monopersulfate gemäß irgendeinem der Ansprüche 1 bis 4 als Bleichmittel in Detergentien-Zubereitungen.

**Patentansprüche für folgenden Vertragsstaat : ES**

1. Verfahren zur Herstellung von Monopersulfaten von Amino-(poly)percarbonsäuren der allgemeinen Formel:

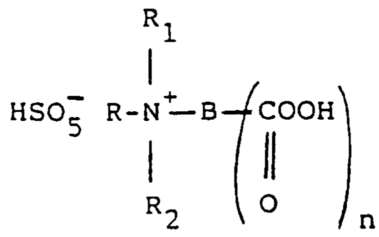

(I)

wobei
R, $R_1$ und $R_2$, die gleich oder von einander verschieden sein können, aus Wasserstoff und wahlweise substituierten Alkylgruppen ausgewählt sind, vorausgesetzt, daß mindestens einer der Reste R, $R_1$ und $R_2$ ein Wasserstoffatom ist;
n eine ganze Zahl von 1 bis 6 bedeutet und B eine Alkylengruppe ist, die gegebenenfalls eingeschobene -$CONR_3$-Einheiten aufweist, wobei $R_3$ ein Wasserstoffatom oder eine Alkyl- oder eine Arylgruppe ist, dadurch gekennzeichnet, daß ein Substrat, das ausgewählt ist aus einer Amino-(poly)carbonsäure und deren N-Sulfat-Salz, die dem gewünschten Monopersulfat der allgemeinen Formel (I) entsprechen, mit $H_2O_2$ in einem Medium aus konzentrierter $H_2SO_4$ umgesetzt wird und daß das daraus resultierende Monopersulfat der allgemeinen Formel (I) durch Zugabe von Tetrahydrofuran und/oder Ethylacetat von der Reaktionsmischung abgetrennt wird.

**2.** Verfahren gemäß Anspruch 1, wobei R, $R_1$ und $R_2$ aus Wasserstoff oder linearen oder verzweigten $C_{1-10}$ Alkylgruppen ausgewählt sind und die Alkylengruppe B von 6 bis 18 Kohlenstoffatome besitzt.

**3.** Verfahren gemäß irgendeinem der Ansprüche 1 und 2, wobei die Alkylengruppe B mindestens eine eingeschobene $CONR_3$-Einheit aufweist, wobei $R_3$ für ein Wasserstoffatom, eine $C_{1-5}$ Alkylgruppe oder eine Arylgruppe steht.

**4.** Verfahren gemäß Anspruch 1, wobei als Monopersulfat der Formel (I) 12-(N,N-Dimethyl-amino)-perlaurinsäure-monopersulfat, 12-Amino-perlaurinsäure-monopersulfat oder 3-Amino-perpropionsäure-monopersulfat hergestellt wird.

**5.** Verfahren gemäß irgendeinem der Ansprüche 1 bis 4, dadurch gekennzeichnet, daß eine Amino-(poly)-carbonsäure, die der gewünschten Verbindung der Formel (I) entspricht, in das entsprechende $HSO_4$-Salz umgewandelt wird, welches anschließend mit $H_2O_2$ in konzentrierter $H_2SO_4$ umgesetzt wird.

**6.** Verfahren gemäß irgendeinem der Ansprüche 1 bis 5, dadurch gekennzeichnet, daß die Amino-(poly)-carbonsäure oder ihr N-Sulfat-Salz schrittweise mit $H_2O_2$ einer Konzentration von 70 Gew.-% bis 90 Gew.-% in konzentrierter $H_2SO_4$ bei einer Temperatur von 0° bis 20°C umgesetzt wird.

**7.** Verfahren gemäß irgendeinem der Ansprüche 1 bis 6, dadurch gekennzeichnet, daß die Menge an $H_2SO_4$ mindestens etwa 5 Mol, vorzugsweise von 6 bis 7 Mol, pro Mol Substrat beträgt.

**8.** Verfahren gemäß irgendeinem der Ansprüche 1 bis 7, dadurch gekennzeichnet, daß mindestens etwa 6 Mol Wasserstoffperoxid, vorzugsweise von 7 bis 10 Mol, pro Mol Substrat verwendet werden.

**9.** Verfahren gemäß irgendeinem der Ansprüche 1 bis 8, dadurch gekennzeichnet, daß das endgültige Mol-Verhältnis von $H_2SO_4$ zum gesamten am Ende der Reaktion anwesenden $H_2O$ im Bereich von 1,2 bis 1,4 liegt.

**10.** Verfahren gemäß irgendeinem der Ansprüche 1 bis 9, dadurch gekennzeichnet, daß die Menge des verwendeten Tetrahydrofurans und/oder Ethylacetats mindestens etwa 4 Liter pro Mol Substrat beträgt.

**11.** Verfahren gemäß irgendeinem der Ansprüche 1 bis 10, dadurch gekennzeichnet, daß das Tetrahydrofuran und/oder Ethylacetat bei einer Temperatur, die 10°C nicht übersteigt, zugegeben wird.

**12.** Verwendung der Monopersulfate gemäß irgendeinem der Ansprüche 1 bis 4 als Bleichmittel in Detergentien-Zubereitungen.

**Revendications**

**Revendications pour les Etats contractants suivants : AT, BE, CH, DE, FR, GB, LI, NL, SE**

**1.** Monopersulfates d'acide amino(poly)percarboxylique répondant à la formule générale (I):

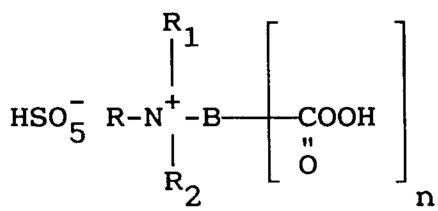

(I)

dans laquelle:

R, $R_1$ et $R_2$, identiques ou différents l'un de l'autre, sont des groupes choisis parmi l'atome d'hydrogène et des radicaux alkyles qui peuvent éventuellement être substitués pourvu qu'au moins l'un desdits groupes R, $R_1$ et $R_2$ soit un atome d'hydrogène;

n est un entier de 1 à 6;

B est un groupe alkylène qui peut éventuellement renfermer un ou plusieurs groupes $-CONR_3$-intercalés, $R_3$ représentant un atome d'hydrogène, un radical alkyle ou un radical aryle.

**2.** Monopersulfates selon la revendication 1, caractérisés en ce que R, $R_1$ et $R_2$ sont choisis parmi l'atome d'hydrogène ou des radicaux alkyles en $C_1$ à $C_{10}$, linéaires ou ramifiés, et le radical alkylène B renferme de 6 à 18 atomes de carbone.

**3.** Monopersulfates selon l'une quelconque des revendications 1 et 2, caractérisés en ce que les radicaux alkylènes B renferment au moins un motif $CONR_3$ intercalé dans lequel $R_3$ représente un atome d'hydrogène, un radical alkyle en $C_1$ à $C_5$ ou un radical aryle.

**4.** Monopersulfates selon la revendication 1, c'est-à-dire monopersulfate d'acide 12-(N,N-diméthylamino)-perlaurique, monosulfate d'acide 12-aminoperlaurique et monopersulfate d'acide 3-aminoperpropionique.

**5.** Procédé de préparation des monopersulfates selon la revendication 1, caractérisés en ce que l'on fait réagir un substrat choisi parmi les acides amino-(poly)carboxyliques ou leurs N-sulfates correspondants, correspondant au monopersulfate recherché de formule (I), réagissant avec $H_2O_2$ dans $H_2SO_4$ concentré, et en ce que le monopersulfate résultant de formule générale (I) est séparé du mélange réactionnel par addition d'un solvant organique consistant en tétrahydrofuranne et/ou acétate d'éthyle.

**6.** Procédé selon la revendication 5, caractérisé en ce qu'un acide amino(poly)carboxylique correspondant au dérivé recherché de formule (I) est converti en sels de $H_2SO_4$ correspondant que l'on fait ensuite réagir avec $H_2O_2$ dans $H_2SO_4$ concentré.

**7.** Procédé selon l'une quelconque des revendications 5 et 6, caractérisé en ce que l'on fait progressivement réagir l'acide amino(poly)carboxylique ou son sel de N-sulfate avec $H_2O_2$ dont la concentration est de 70 à 90% en poids dans $H_2SO_4$ concentré à une température de 0 °C à 20 °C.

**8.** Procédé selon l'une quelconque des revendications 5 à 7, caractérisé en ce que la quantité de $H_2SO_4$ est au moins égale à environ 5 moles et qu'elle est de préférence comprise entre 6 et 7 moles par mole de substrat.

**9.** Procédé selon l'une quelconque des revendications 5 à 8, caractérisé en ce que le peroxyde d'hydrogène est utilisé en quantité d'au moins 6 moles, et de préférence de 7 à 10 moles par mole de substrat.

10. Procédé selon l'une quelconque des revendications 5 à 9, caractérisé en ce que le rapport molaire final de $H_2SO_4/H_2O$ totale présent à la fin de la réaction est compris entre 1,2 et 1,4.

11. Procédé selon l'une quelconque des revendications 5 à 10, caractérisé en ce que la quantité de tétrahydrofuranne et/ou d'acétate d'éthyle utilisé n'est pas inférieure à environ 4 litres par mole de substrat.

12. Procédé selon l'une quelconque des revendications 5 à 11, caractérisé en ce que le tétrahydrofuranne et/ou l'acétate d'éthyle servant de solvant est ajouté à une température ne dépassant pas environ 10 °C.

13. Utilisation des monopersulfates l'une quelconque des revendications 1 à 4, comme agents de blanchiment dans des formulations détergentes.

**Revendications pour l'Etat contractant suivant : ES**

1. Procédé de préparation de monopersulfates d'acide amino(poly)percarboxyliques répondant à la formule générale (I):

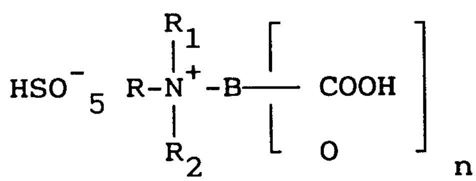

(I)

dans laquelle:

R, $R_1$ et $R_2$, identiques ou différents l'un de l'autre, sont choisis parmi l'hydrogène et des groupes alkyles éventuellement substitués pourvu qu'au moins l'un d'entre R, $R_1$ et $R_2$ soit un atome d'hydrogène;

n est un nombre entier de 1 à 6; et

B est un groupe alkylène renfermant éventuellement des motifs $-CONR_3-$ intercalés, dans lesquels $R_3$ représente un atome d'hydrogène ou un groupe alkyle ou aryle, caractérisé en ce que l'on fait réagir un substrat choisi parmi les acides amino-(poly)carboxyliques ou leurs N-sulfates correspondants, correspondant au monopersulfate recherché de formule (I), avec $H_2O_2$ dans $H_2SO_4$ concentré, et en que le monopersulfate résultant de formule générale (I) est séparé du mélange réactionnel par addition de tétrahydrofuranne et/ou acétate d'éthyle.

2. Procédé selon la revendication 1, caractérisés en ce que R, $R_1$ et $R_2$ sont choisis parmi l'hydrogène ou des groupes alkyles en $C_1$ à $C_{10}$, linéaires ou ramifiés, et le groupe alkylène B renferme de 6 à 18 atomes de carbone.

3. Procédé selon l'une quelconque des revendications 1 et 2, caractérisés en ce que les groupes alkylènes B renferment au moins un motif $CONR_3$ intercalé dans lequel $R_3$ représente un atome d'hydrogène, un groupe alkyle en $C_1$ à $C_5$ ou un groupe aryle.

4. Procédé selon la revendication 1, caractérisé en ce que l'on préparé en tant que monopersulfate de formule (I):
monopersulfate d'acide 12-(N,N-diméthylamino)perlaurique, monosulfate d'acide 12-aminoperlaurique ou monopersulfate d'acide 3-aminoperpropionique.

5. Procédé selon l'une quelconque des revendications 1 à 4, caractérisé en ce qu'un acide amino(poly)-carboxylique correspondant aux dérivé recherché de formule (I) est converti en sels de $H_2SO_4$ correspondant que l'on fait ensuite réagir avec $H_2O_2$ dans $H_2SO_4$ concentré.

6. Procédé selon l'une quelconque des revendications 1 et 5, caractérisé en ce que l'on fait progressivement réagir l'acide amino(poly)carboxylique ou son sel de N-sulfate avec $H_2O_2$ dont la concentration est de 70 à 90% en poids dans $H_2SO_4$ concentré à une température de 0 ° C à 20 ° C.

7. Procédé selon l'une quelconque des revendications 1 à 6, caractérisé en ce que la quantité de $H_2SO_4$ est au moins égale à environ 5 moles et qu'elle est de préférence comprise entre 6 et 7 moles par mole de substrat.

8. Procédé selon l'une quelconque des revendications 1 à 7, caractérisé en ce que le peroxyde d'hydrogène est utilisé en quantité d'au moins 6 moles et de préférence de 7 à 10 moles par mole de substrat.

9. Procédé selon l'une quelconque des revendications 1 à 8, caractérisé en ce que le rapport molaire final de $H_2SO_4$ sur le total de $H_2O$ présent à la fin de la réaction est compris entre 1,2 et 1,4.

10. Procédé selon l'une quelconque des revendications 1 à 9, caractérisé en ce que la quantité de tétrahydrofuranne et/ou d'acétate d'éthyle utilisé n'est pas inférieure à environ 4 litres par mole de substrat.

11. Procédé selon l'une quelconque des revendications 1 à 10, caractérisé en ce que le tétrahydrofuranne et/ou l'acétate d'éthyle servant de solvant est ajouté à une température ne dépassant pas environ 10 ° C.

12. Utilisation des monopersulfates l'une quelconque des revendications 1 à 4, comme agents de blanchiment dans des formulations détergentes.